## Europäisches Patentamt
## European Patent Office
## Office européen des brevets

(19)

(11) Numéro de publication: **0 080 417 B1**

(12) # FASCICULE DE BREVET EUROPEEN

(45) Date de publication du fascicule du brevet:
**28.10.87**

(51) Int. Cl.⁴: **A 61 B 10/00**, A 61 B 5/10

(21) Numéro de dépôt: **82402125.7**

(22) Date de dépôt: **22.11.82**

(54) **Procédé pour l'enregistrement de mouvements foetaux au cours de la grossesse et appareil pour la mise en oeuvre du procédé.**

(30) Priorité: **20.11.81 FR 8121823**

(43) Date de publication de la demande:
**01.06.83 Bulletin 83/22**

(45) Mention de la délivrance du brevet:
**28.10.87 Bulletin 87/44**

(84) Etats contractants désignés:
**AT BE CH DE GB IT LI LU NL SE**

(56) Documents cités:
**AT - A - 310 929**
**DE - A - 2 424 658**
**FR - A - 2 045 857**
**FR - A - 2 091 933**
**FR - A - 2 306 455**
**US - A - 3 451 260**
**US - A - 3 606 879**

(73) Titulaire: **UNIVERSITE DE FRANCHE-COMTE, 30 Avenue de l'Observatoire, F-25030 Besançon (FR)**

(72) Inventeur: **Brandon, Jean-François, 7 Square St. Amour, F-25000 Besancon (FR)**
Inventeur: **Colette, Claude, Rond Point Carnot, F-25000 Besancon (FR)**
Inventeur: **Fabbri, Marcel, 32 rue Gabriel Plançon, F-25000 Besancon (FR)**
Inventeur: **Weichlein, Bernard, 11 rue Danton, F-25000 Besancon (FR)**

(74) Mandataire: **Beauchamps, Georges et al, Cabinet Z.Weinstein 20, avenue de Friedland, F-75008 Paris (FR)**

## Description

La présente invention a pour objet un appareil pour l'enregistrement des mouvements fœtaux au cours de la grossesse, selon le préambule de la revendication principale.

Un appareil de ce type est connu par la demande de brevet français N° 2 045 857. Cet appareil connu est conçu pour permettre d'assurer une mesure exacte et directionnelle continue de la vitesse et du déplacement d'une surface en mouvement, par exemple du mouvement d'un fœtus.

Cet appareil connu a pour inconvénient majeur de présenter une structure relativement complexe comprenant un dispositif émetteur d'ondes ultrasonores qui fonctionne dans le mode d'émission continu et un dispositif récepteur-analyseur adapté pour détecter l'information de modulation de fréquence et de modulation d'amplitude contenue dans le signal Doppler et pour effectuer une division analogique de l'information de fréquence détectée par l'information de modulation d'amplitude de manière à obtenir un signal de sortie représentatif de la vitesse de la surface. Pour obtenir le déplacement en fonction du temps, on prévoit un circuit destiné à intégrer simultanément la fonction de vitesse. La complexité de la structure de cet appareil connu s'explique par les informations très spécifiques qu'il doit donner, relatives à la surface en mouvement. L'appareil est conçu pour être utilisé dans des institutions spécifiques, tels que des hôpitaux, par des personnes spécialisées tels que des médecins.

D'autres appareils permettant la détection du mouvement de surfaces réfléchissantes sont décrits dans le brevet autrichien N° 310 929 et le brevet US N° 3 606 879. Ils ne sont pas décrits comme étant utilisables pour la détection des mouvements d'un fœtus. De façon générale, ils sont conçus pour permettre une surveillance épisodique d'un phénomène dans le but de faire un diagnostic. Selon le brevet US, on surveille certains phénomènes physiologiques au cours de la naissance. Dans le cas du brevet autrichien, on surveille le mouvement de faisceaux sanguins et du cœur d'un malade. Dans tous les cas, les résultats de la surveillance nécessitent l'intervention d'un spécialiste, c'est-à-dire d'un médecin, pour pouvoir interpréter. De ce fait, seulement le transducteur est appliqué à la peau de la personne à surveiller, tandis que le système analyseur est réalisé sous forme d'une unité séparée.

Par rapport aux appareils connus, le problème de l'invention consiste à proposer un appareil qui est adapté pour permettre une surveillance permanente d'un phénomène physiologique, dans des conditions de vie normale, sans que la présence d'un spécialiste soit nécessaire. Un tel appareil doit présenter une structure très simple et afficher le résultat de la détection sous une forme facile à interpréter par un non-spécialiste telle que la mère.

Pour atteindre ce but, l'appareil selon l'invention est conçu de la manière indiquée dans la partie caractérisante de la revendication principale.

En fonctionnant en mode d'émission pulsée, l'appareil selon l'invention accomplit une sélection dans le temps des signaux réfléchis à la suite de la détermination d'une période de réception. Cette période permet d'éliminer d'emblée, avant l'analyse proprement dite, les signaux parasites provenant d'autres surfaces réfléchissantes. Le mode d'émission pulsée et l'utilisation d'un détecteur du début et de la fin du mouvement d'un fœtus et d'un compteur incrémenté par le signal indicateur d'un mouvement permettent de réaliser un appareil présentant une structure simple et peu encombrante permettant de loger l'ensemble dans un boîtier portable par la mère.

L'invention sera mieux comprise, et d'autres buts, caractéristiques, détails et avantages de celle-ci apparaîtront plus clairement au cours de la description explicative qui va suivre faite en référence aux dessins schématiques annexés donnés uniquement à titre d'exemple illustrant un mode de réalisation de l'invention, et dans lesquels:

La figure 1 est une vue en perspective de l'appareil d'enregistrement des mouvements fœtaux, selon la présente invention.

La figure 2 montre, sous forme d'un schéma bloc, la structure du montage électrique de l'appareil suivant la figure 1.

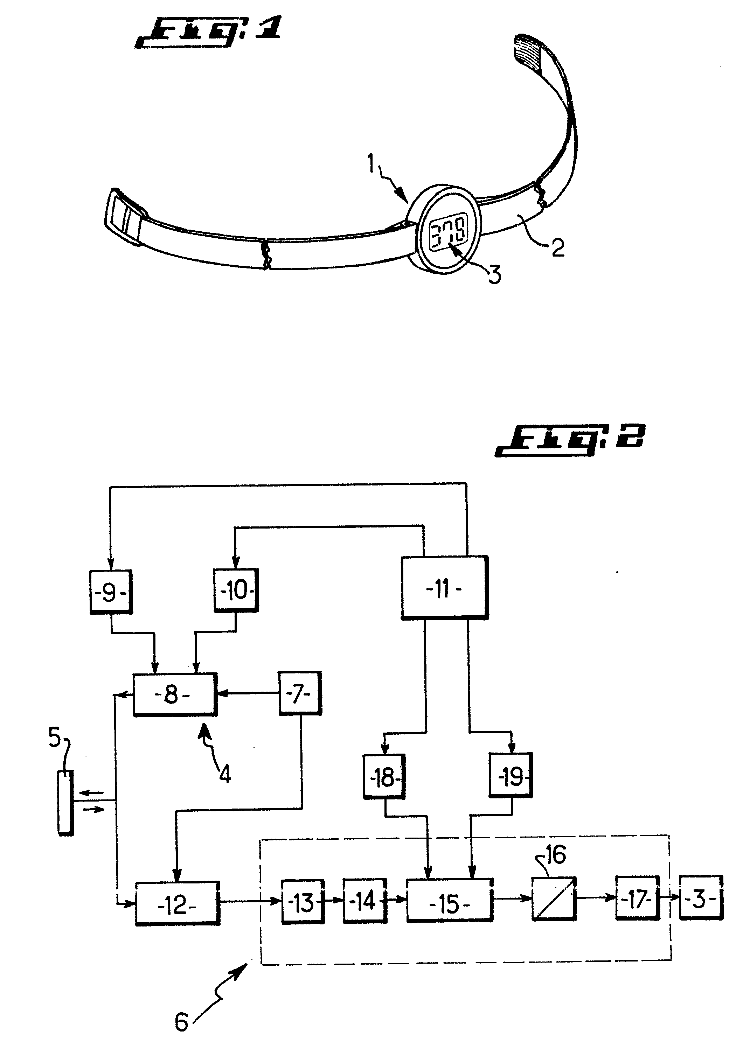

Comme l'illustre la figure 1, tous les dispositifs du système électronique de l'appareil d'enregistrement et de comptage des mouvements fœtaux, selon l'invention, sont enfermés dans un boîtier 1, par exemple de forme cylindrique et de dimensions appropriées pour qu'il soit portable en étant appliqué à la surface de la peau abdominale, de préférence, près de l'ombilic. Sa mise en place s'effectue, soit interposé entre la ceinture de grossesse et l'abdomen, soit à l'aide d'une sangle 2 en cas d'absence d'une telle ceinture. Le boîtier 1 est pourvu d'un afficheur numérique 3, par exemple à diodes électroluminescentes ou à cristaux liquides. L'afficheur a pour fonction de permettre la visualisation à tout instant du nombre de mouvements fœtaux. Suivant la figure 1, il est prévu sur la surface supérieure du boîtier mais pourrait être situé sur la tranche de celui-ci. Il est encore possible de réaliser l'afficheur par un cadran gradué et une aiguille. Le boîtier peut avoir un diamètre de l'ordre de 8 cm et une hauteur de 2 cm. Ces valeurs ne sont données qu'à titre d'exemple et ne sont pas limitatives.

Suivant le schéma bloc de la figure 2, le système électronique enfermé dans le boîtier 1 comprend un dispositif émetteur d'ondes électriques 4, un dispositif transducteur ultra-sonore 5 et un dispositif récepteur et analyseur 6 des ondes obtenues des ondes ultra-sonores réfléchies par le fœtus. Le transducteur fonctionne avantageusement dans la gamme des fréquences 1 à 10 MHz, qui est la gamme de fréquence la mieux adaptée au corps humain.

Le dispositif émetteur 4 comporte un oscillateur 7 et un émetteur 8 reliés à l'oscillateur 7 et commandé par des circuits de commande du début d'émission 9 et de la durée d'émission 10, sous l'effet d'une base de temps 11. On obtient ainsi une émission pulsée d'ondes ultra-sonores.

Le système récepteur-analyseur 6 comprend, en série, un élément récepteur 12, un filtre 13 accordé sur une bande de fréquences, un amplificateur 14, un dispositif formant une porte commandée 15, un détecteur de mouvements fœtaux 16, un compteur 17 et le dispositif afficheur 3 déjà mentionné lors de la description de la figure 1. Le compteur 17 est susceptible d'être remis à zéro. Le dispositif 15 formant porte est commandé par un circuit de commande d'ouverture 18 et ainsi du début de l'observation et par un circuit de commande 19 de la durée d'ouverture ou d'observation, tous les deux sous l'effet de la base de temps 11. Le détecteur 16 est agencé pour détecter le début et la fin d'un mouvement du fœtus. Cette double fonction est illustrée par le partage en deux du bloc 16. C'est la détection de la fin du mouvement qui commande l'incrémentation du compteur 17. Ceci signifie qu'au cours d'un mouvement ou d'une phase de mouvements le compteur ne fait qu'un seul pas en avant. Le filtre 13 est conçu pour éliminer toutes les ondes provoquées par des échos parasites.

Les durées de temps d'émission et d'observation, déterminées respectivement par les circuits 10 et 19, ainsi que les intervalles de temps entre, d'une part, la fin d'une émission d'ondes ultra-sonores et l'ouverture de la porte 15, et, d'autre part, la fermeture de cette porte 15 et le début de la prochaine émission d'ondes ultra-sonores sont réglables pour permettre une adaptation de l'appareil aux particularités du comportement de chaque fœtus.

Le dispositif transducteur 5 peut être formé par deux transducteurs, un pour l'émission et l'autre pour la réception. Mais il peut être constitué par un seul transducteur accomplissant les deux fonctions.

Selon la présente invention, le transducteur 5 qui est placé de préférence sur la surface de la peau abdominale de la mère, par exemple près de l'ombilic, émet de façon pulsée des ondes ultra-sonores. L'émission pulsée a pour avantage d'éliminer les ambiguïtés de distance en évitant les interférences des structures situées en amont ou en aval du volume en mouvement. La détection d'un mouvement du fœtus est fondée sur l'analyse des ondes réfléchies par celui-ci.

L'analyse fréquentielle exploite l'effet Doppler; à savoir, le changement de fréquence que subissent les ondes réfléchies lorsque le fœtus bouge, qui peut être considéré comme une surface réfléchissante en déplacement. La différence entre la fréquence des ondes émises et des ondes réfléchies est proportionnelle à la vitesse du déplacement de la surface, c'est-à-dire, du mouvement du fœtus. Il s'est avéré que les mouvements du fœtus que l'on désire détecter sont de l'ordre de plusieurs 100 Hz. Par exemple, pour une fréquence d'émission de 5 MHz et un déplacement à une vitesse de 10 cm/sec, on obtient un changement de fréquence d'environ 650 Hz. Par contre, les changements de fréquence provoqués par des déplacements parasites de surfaces réfléchissantes, tels que par exemple produits par la respiration de la mère et du mouvement cardiaque du fœtus, sont en général des déplacement à faible vitesse et occasionnent des fréquences de différence plus faibles, inférieures à 10 ou 20 Hz. Dans ces conditions, en fixant la fréquence limite inférieure du filtre 13 à une valeur appropriée, par exemple à quelques dizaines de Hz, les déplacements parasites peuvent être sûrement éliminés. Un choix approprié des intervalles de temps susmentionnés, d'une part, entre l'émission d'un impulsion d'ondes ultra-sonores et la période d'observation, et, d'autre part, entre celle-ci et l'émission de la prochaine impulsion, contribue à une bonne sélectivité du dispositif analyseur selon l'invention. Il est à noter que la ré-émission d'ondes ultra-sonores, après la détection du début d'un mouvement du fœtus doit être assurée pour que l'appareil puisse détecter la fin du mouvement, détection qui donne lieu à l'incrémentation du compteur 17. Les intervalles de temps peuvent être réglés à l'aide des circuits 9 et 18. L'intervalle de temps entre l'émission d'une impulsion d'ondes ultra-sonores et le début de la phase d'observation des ondes réfléchies, c'est-à-dire de l'ouverture de la porte 15 tient compte de la distance du fœtus du transducteur et du temps qu'il faut aux ondes ultra-sonores pour parcourir la distance du transducteur au fœtus et de celui-ci au transducteur. Il est évident que cet intervalle de temps doit être réglable pour tenir compte des particularités de chaque cas d'application.

Conformément à l'invention, il est possible de remplacer le système analyseur, qui vient d'être décrit, c'est-à-dire, la partie qui se trouve à l'intérieur de la ligne interrompue de la figure 2, à l'exception par exemple de l'amplificateur mais en y incluant les circuits 9, 10, 18 et 19 par un micro-processeur. Celui-ci peut être programmé pour chaque cas d'application. Ainsi les fréquences limites du filtre, les fréquences d'analyse et les intervalles de temps comme les durées d'émission et d'observation peuvent être programmées. L'appareil selon l'invention, équipé d'un micro-processeur peut donner des informations plus complexes et par exemple permettre la gestion de l'afficheur 3 et la mémorisation et la gestion de l'histogramme des événements. Il serait ainsi possible de connaître le temps qui s'est écoulé entre deux mouvements du fœtus et éventuellement la vitesse des mouvements. La programmation peut être faite par exemple par un programmeur, à l'aide d'un clavier alpha-numérique. Les déplacements considérés comme des déplacements parasites, tels que la respiration de la mère et les mouvements cardiaques du fœtus peuvent être enregistrés dans une mémoire et consituer des valeurs de référence. En comparant successivement les signaux reçus aux signaux de référence mémorisés, le microprocesseur pourrait reconnaître les signaux provoqués par un mouvement du fœtus.

0 080 417

Il est encore à noter que dans le cas de l'analyse fréquentielle, le récepteur 12 a pour fonction particulière de produire à sa sortie des signaux de basse fréquence obtenus par comparaison des ondes émises et des ondes réfléchies. A cette fin, le récepteur 12 est relié à l'oscillateur 7. Il pourrait par exemple comprendre un étage mélangeur. Le filtre 13 est donc dans ce cas accordé à une bande de basses fréquences, comme cela a été expliqué plus haut.

Bien entendu, de nombreuses modifications peuvent être apportées à l'invention sans quitter le cadre de celle-ci- Par exemple, au lieu de prévoir un analyseur, on pourrait remplacer celui-ci par un indicateur acoustique, tel qu'un microphone. Cet indicateur sera monté en aval du filtre. Il est encore à noter que l'invention pourrait être utilisée pour la détection du mouvement d'autres volumes qu'un fœtus. Il pourrait être utilisé pour la détection du mouvement du cœur.

**Revendications**

1. Appareil pour l'enregistrement des mouvements fœtaux au cours de la grossesse, comprenant un dispositif transducteur (5) ultra-sonore relié, d'une part, à un dispositif émetteur d'ondes électriques (7, 8), pour émettre des ondes ultra-sonores en direction du fœtus, et, d'autre part, à un dispositif récepteur (12) des ondes électriques obtenues à parties des ondes ultra-sonores, qui ont été réfléchies par le fœtus et reçues par le transducteur (5), et un dispositif d'analyse (13, 17) desdits signaux électriques, le dispositif d'analyse comprenant un moyen détecteur (15, 16) de la présence ou absence de signaux électriques représentatifs d'un mouvement du fœtus pendant des périodes de réception prédéterminées, caractérisé par un émetteur adapté pour émettre les ondes ultra-sonores de façon pulsée, par un détecteur (16) adapté pour détecter le début et la fin d'un mouvement du fœtus et par un compteur (17) dont l'entrée est reliée à la sortie du détecteur (16, la détection d'un mouvement du fœtus par le détecteur (16) constituant un signal d'incrémentation du compteur (17) et en ce que le transducteur (5), le dispositif émetteur (4) et le dispositif récepteur-analyseur (6) et le compteur (17) sont montés dans un boîtier (1) portable en étant appliqué sur la surface de la peau abdominale de la mère.

2. Appareil selon la revendication 1, caractérisé en ce que le boîtier (1) est pourvu d'un sangle (2) de mise et maintien en place de l'appareil.

**Claims**

1. Apparatus for recording foetal movements during pregnancy, comprising an ultrasonic transductor device (5) coupled on the one hand to an electric wave emitting device (7, 8) for emitting ultrasonic waves towards the foetus, and, on the other hand, a receiver device (12) for receiving the electric waves developed from the ultrasonic waves, which have been reflected by the foetus and received by the transductor (5), and an analysing device (13, 17) of said electrical signals, the analysing device comprising detector means (15, 16) for detecting the presence or absence of electrical signals representative of a foetal movement during predetermined receiving periods, characterized by an emitter adapted for emitting ultrasonic waves in a pulsed fashion, by a detector (16) adapted to detect the beginning and the end of a movement of the foetus and by a counter (17) the input of which is coupled to the output of the detector (16), the detection of a foetal movement by the detector (16) constituting an incrementing signal for the counter (17), and in that the transductor (5), the emitter device (4) and the receiver-analyser device (6) and the counter (17) are mounted in a casing (1) which can be carried in a condition applied to the surface of the abdomen skin of the mother.

2. Apparatus according to claim 1, characterized in that the casing (1) is provided with a strap (2) for putting and holding said apparatus in place.

**Patentansprüche**

1. Gerät zur Aufzeichnung von Foetalbewegungen während der Schwangerschaft, mit einer Ultraschalltransductorvorrichtung (5), die einerseits mit einer elektrische Wellen aussendenden Vorrichtung (7, 8) zur Aussendung von Ultraschallwellen in Richtung zum Foetus hin und andererseits einer Vorrichtung (12) zum Empfang der von Ultraschallwellen erhaltenen elektrischen Wellen verbunden ist, die von dem Foetus reflektiert worden und von dem Transductor (5) empfangen worden sind, und mit einer Vorrichtung (13, 17) zur Analyse der genannten elektrischen Signale, wobei die Analysevorrichtung Detektormittel (15, 16) für das Vorhandensein oder die Abwesenheit von elektrischen Signalen besitzt, die eine Bewegung des Foetus während bestimmter Empfangsperioden bedeuten, gekennzeichnet durch einen Sender, der geeignet ist, Ultraschallwellen in pulsierender Form auzusenden, durch einen Detektor (16), der geeignet ist, den Beginn und das Ende einer Bewegung des Foetus zu erkennen, und durch einen Zähler (17), dessen Eingang mit dem Ausgang des Detektors (16) verbunden ist, wobei die Erkennung einer Bewegung des Foetus durch den Detektor (16) ein Signal zur Inkrementierung des Zählers (17) darstellt, und dadurch gekennzeichnet, dass der Transductor (5), die Sendervorrichtung (4) und die Empfängeranalysiervorrichtung (6) und der Zähler (17) in ein Gehäuse (1) eingebaut sind, das in auf die Oberfläche der Bauchhaut der Mutter aufgelegter Weise getragen werden kann.

2. Gerät nach Anspruch 1, dadurch gekennzeichnet, dass das Gehäuse (1) mit einem Gut (2) zum Anbringen und zur Halterung des Gerätes versehen ist.

**Fig. 1**

**Fig. 2**